# EUROPEAN PATENT APPLICATION

(11) **EP 1 304 047 A1**
(43) Date of publication of application: **23.04.2003**
(21) Application number: 02015602.2
(22) Date of filing: 15.07.2002
(51) Int. Cl.: A23L 1/30

(54) **Extracts of cacao bean and cacao bean husk with inhibitory effects on carcinogenesis**

(30) Priority: 18.10.2001 KR 2001064285
(71) Applicant: LOTTE CONFECTIONERY CO., Ltd., Seoul 150-964 (KR)
(72) Inventor: Lee, Hyong Joo, Seocho-ku, 137-754 Seoul (KR); Lee, Ki Won, 441-100 Suwon, Kyunggido (KR); Kang, Kyung Sun, Jangan-ku, 440-300 Suwon, Kyunggi-do (KR); Kim, Dong Young, 423-033 Gwangmyung-shi, Kyunnggi-do (KR); Park, Hyung Hwan, 801-502 Shinan Apt.,, 412-725 Goyang-shi, Kyunggi-do (KR); Lee, Man Jong, 104-1104 Doosandonga Apt.,, 441-390 Suwon, Kyunggi-do (KR); Kim, Han Soo, 310-704 Gangchon-maeul, 411-350 Goyang-shi, Kyunggi-do (KR); Kwon, Ik Boo, 2404 LotteGwank Tower,, 156-010 Seoul (KR)
(74) Representative: Reinhard - Skuhra - Weise & Partner

(57) **Abstract**

The present invention relates to extract and fraction of cacao bean and cacao bean husk, which inhibit the suppression of GJIC (gap junctional intercellular communication) and inhibit DNA synthesis of cancer cell, pathological phenomena occurring during promotion and progression stages of carcinogenesis.

## Description

### FIELD OF THE INVENTION

The present invention relates to the extracts of cacao bean and cacao bean husk with inhibitory effects on carcinogenesis, and more particularly, to extracts and fractions of cacao bean and cacao bean husk which inhibit the suppression of gap junctional intercellular communication (GJIC), a pathological phenomenon occurring during the development of various kinds of cancers including liver cancer, as well as DNA synthesis of cancer cells thereby inhibiting proliferation of cancer cells.

### BACKGROUND OF THE INVENTION

Cacao (Theobroma cacao L.), an active ingredient of chocolate and cocoa, denotes a mysterious meaning of God's food. The place of origin of cacao appears to be some areas ranging from Central America to the northern parts of South America. Several countries in Central America have regarded cacao as one of the valuable foods along with corn, which has a long history of cultivation since the prehistoric times, and it is now widely beloved by the majority of people around the world regardless of race, age and sex.

Cacao bean, an active ingredient of chocolate, commonly contains a large amount of phenolic phytochemicals and theobromine as well as dietary fibers, although its composition varies depending on where it is cultivated. During the manufacture of chocolate, the nib portion of peeled-off cacao bean is used as a raw material for chocolate or cocoa, whereas most of its husks (about 15 wt% of the total weight of cacao bean) generated as a by-product, is subsequently discarded. For example, the current worldwide production of cacao bean is about 2.5 million tons, of which about 400,000 tons of cacao bean husk is being discarded. According to the annual trade statistics released by the Office of Customs Administration of Korea in 1990, about 600 tons of cacao bean husk had been abandoned as wastes in Korea. However, the cacao bean husk is now known to contain a large amount of phenolic phytochemicals and theobromine as well as dietary fibers as is the case with the cacao bean.

From now on, the research on the functional utilization of the cacao bean has first been focusing on the polyphenols, procyanidin in cacao bean. **The inventors** (**Mars Inc.**) previously disclosed that only procyanidin fractions of cacao bean are able to extirpate cancerous cells of leukemia, prostate cancer, colon cancer, lung cancer, breast cancer and the like as well as inhibit the activity of topoisomerase II (Korea Patent Application No. 1998-707952). However, the inventors have not shown the effects of cacao bean on liver and gastric cancers, and also the fraction containing theobromine in the above patent, unlike the fraction containing procyanidins, had no effects against cancer.

The inventors of the present invention before the invention above, disclosed that cacao bean husk obtained from the manufacture of chocolate as a by-product is economically feasible and its chemical polymer composition containing procyanidin A and procyanidin C-1 is effective in the inhibition of glucosyltransferase, a major cause of tooth decay, thus preparing a chewing gum designed to prevent tooth decay by blending a soluble extract of cacao bean husk (Korea Pat. No. 1991-45179 and U.S. Pat. No. 4,908,212).

The inventors of the present invention previously also discloses a method to manufacture a fraction of cacao bean husk with an improved inhibitory activity of glucosyltransferase by increasing the amount of polyphenol content (U.S. Pat. No. 6,159,451 ).

As descried above, cacao bean and cacao bean husk contain a variety of physiologically active components such as dietary fibers, phenolic phytochemicals and theobromine. Therefore, the physiological activity of cacao bean and cacao bean husk cannot be solely represented by a certain structure of procyanidin alone and also the commercial utilization of the certain structure of procyanidin does not appear to be feasible because of high production cost. Therefore, it would be more practicable to utilize the fraction having many improved physiological activities prepared by a simple separation process as disclosed in U.S. Pat. No. 6,159,451.

Despite surgical operations, radiation therapies and chemotherapies for the past few decades, the rates of occurrence of most epithelial cell cancers including liver cancer, stomach cancer and colon cancer and the mortality rate resulted from the above cancers were not significantly reduced (Sporn, M. B., Lancet, 347:1377-1381, 1996). This appears due to the fact that the approaches on cancers for the past thirty years have been mostly focused on a therapeutic point rather than a preventive point. As is already known, intake of certain food or a drug that contains components which can effectively inhibit or delay the multi-step progress of cancer will help to reduce the risk of cancer and the subsequent mortalities resulted thereof and numerous researches have been carried out (Kang et al., *Chemcial prevention of cancer,* Korea Medicine, 2000; Surh, Y. J., *Mutat. Res,* 428:305-327, 1999; Sporn, M. B., Lancet, 347:1377-1381, 1996; Caragay, A. B., *Food Technol.,* 65-68, 1992).

Carcinogenesis is a multi-step process consisting of three stages of initiation, promotion and progression. In searching of agents which may be effective in prevention or inhibition of cancer, the recent researches have been more concerned with identifying substances that can inhibit promotion and progression stages rather than those of initiation stage, which is a short-term and irreversible stage (Kang et al., *Chemoprevention of cancer,* Korea Medicine, 2000; Surh, Y. J., *Mutat. Res,* 428: 305-327, 1999; Sporn, M. B., *Lancet,* 347: 1377-1381, 1996). In particular, unlike pharmaceutical drugs, the prevention and inhibition of cancer by means of food extract and fraction will become more effective if they target on the promotion stage of carcinogenesis, which is generally progressed for more than 20 years and is also reversible (Kang et al., *Chemoprevention of cancer,* Korea Medicine, 135-136, 2000; Yamasaki, H. et al, *Carcinogenesis* 11: 1051-1058, 1990; Kelloff. G. J. et al., *Eur. J. Cancer,* 35: 1755-1762, 1999; Surh, Y. J., *Mutat. Res,* 428: 305-327, 1999). Gap junctional intercellular communication (GJIC) is essential for the modulation of the homeostatic balance through the modulation of cell proliferation and differentiation in multicellular organisms. Inhibition of GJIC is considered as a key biochemical index observed at carcinogenesis, particularly to cancer promotion stage; therefore, substances that can inhibit such a process is expected to inhibit the promotion stage of carcinogenesis thereby preventing as well as inhibiting the development of cancer. Further, proliferation of cancer cells by DNA synthesis is considered as a key biological index observed at the progression stage of carcinogenesis; therefore, substances that can inhibit such a process is expected to inhibit the progression stage of carcinogenesis thereby preventing as well as inhibiting the development of cancer (Kang et al., *Chemoprevention of cancer,* Korea Medicine, 135-136, 2000; Yamasaki, H. et al, *Carcinogenesis* 11:1051-1058,1990; Kelloff. G. J. et al., *Eur. J. Cancer,* 35: 1755-1762, 1999; Surh, Y. J., *Mutat. Res,* 428: 305-327, 1999; Holder. J. W. et al, *Cancer Res.,* 53, 3475-3485, 1993).

### SUMMARY OF THE INVENTION

The inventors of the present invention carried out extensive studies in search of a promising candidate for an anti-carcinogenic agent from natural foods for the safety reason and found that the extract of cacao bean and cacao bean husk has an anti-carcinogenic activity such as inhibiting the suppression of GJIC occurring during promotion stage of carcinogenesis, playing an important role in homeostasis of a body, and inhibiting the proliferation via inhibiting the DNA synthesis of cancer cells. Further, the fraction of cacao bean and cacao bean husk obtained by preparing the extract of cacao bean and cacao bean husk by solvent extraction, filling it into an adsorption resin, fractioning with 40% ethanol and re-fractioning the remnant with 60% ethanol, has excellent inhibitory effect on the development of cancer. Also, utilization of cacao bean husk which had been discarded as wastes can increase the economic values.

Therefore, the object of the present invention is to provide an anti-carcinogenic agent comprising extract and fraction of cacao bean and cacao bean husk as an active ingredient.

### BRIEF DESCRIPTION OF THE DRAWINGS

FIG. 1 is a set of pictures that show inhibitory effect of the fraction of cacao bean and cacao bean husk against suppression of GJIC generated by H₂O₂, a cancer promoter and one of the strongest reactive oxygen species (ROS) in the human body, in rat liver epithelial cells (a: a control group; b: a group treated with 400 µM H₂O₂, c: a group treated with 10 µg/mL cacao bean fraction + 400 µM H₂O₂, d: a group treated with 10 µg/mL cacao bean husk fraction + 400 µM H₂O₂).
FIG. 2 is a picture that shows inhibitory effect of cacao bean and cacao bean husk against hyperphosphorylation of connexin 43, a major protein that controls GJIC, generated by H₂O₂, in rat liver epithelial cells (a: a control group; b: a group treated with 400 µM H₂O₂, c: a group treated with 10 µg/mL cacao bean fraction + 400 µM H₂O₂, d: a group treated with 10 µg/mL cacao bean husk fraction + 400 µM H₂O₂).

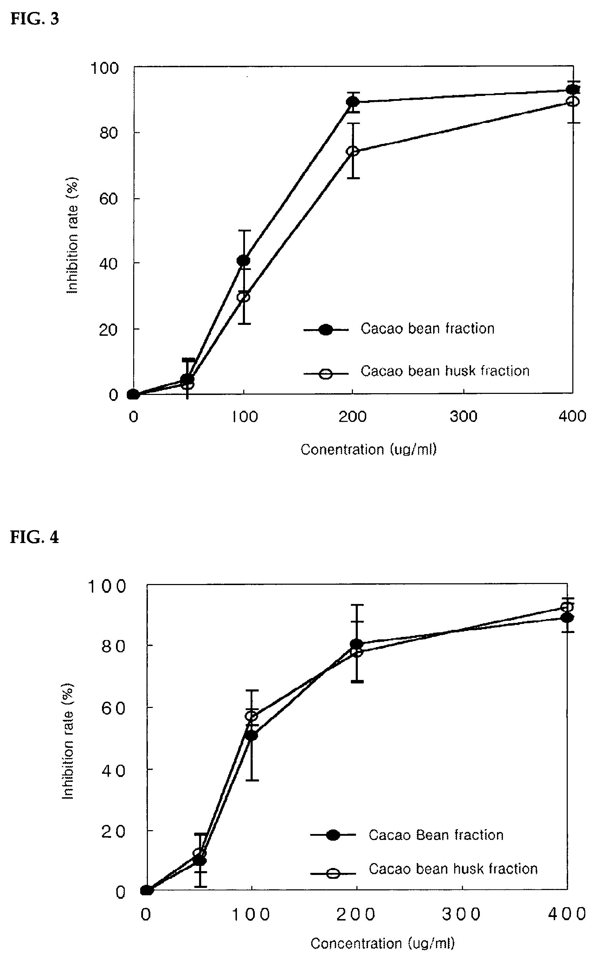

FIG. 3 is a graph that shows inhibitory effect of the fraction of cacao bean and cacao bean husk against liver cancer cell proliferation via inhibition of DNA synthesis of HepG2, a liver cancer cell.
FIG. 4 is a graph that shows inhibitory effect of the fraction of cacao bean and cacao bean husk against liver cancer cell proliferation via inhibition of DNA synthesis of SNU1, a gastric cancer cell.
FIG. 5 is a graph that shows inhibitory effect of the fraction of cacao bean and cacao bean husk against liver cancer cell proliferation via inhibition of DNA synthesis of SNUC2A, a colon cancer cell.

### DETAILED DESCRIPTION OF THE INVENTION

The present invention relates to an anti-carcinogenic agent comprising extract and fraction of cacao bean and cacao bean husk, as an active ingredient.

The present invention is described in more detail as set forth hereunder.

The extract and fraction of cacao bean and cacao bean husk of the present invention is useful in prevention as well as inhibition of carcinogenesis by not only inhibiting the suppression of GJIC but also inhibiting DNA synthesis of cancer cells, which are characteristic phenomena occurring during the promotion and progression stages of carcinogenesis. Therefore, the present invention also relates to a pharmaceutical drug or a food additive that contains extract and fraction of cacao bean and cacao bean husk as an active ingredient.

The process of manufacturing extract and fraction from cacao bean and cacao bean husk is as follows.

First, cocoa butter is removed from dried cacao bean and 1 part by wt of the remaining cacao bean fraction (cacao mass) or cacao bean husk is added with 4-10 parts by wt of aqueous solution of 50% acetone, 50% ethanol or 50% methanol. Then, agitation extraction is performed for the mixture while refluxing for 4-6 hr at 40-70 °C. Supernatant is recovered after centrifugation of the resulting extract, and the remaining mass is further extracted by repetition. The extract is dried, filtered, and then the extract of cacao bean and cacao bean husk is obtained. Thus obtained extract is adsorbed to styrene-based porous resin, washed with 40% ethanol, and then solvent-fractioned with 60% ethanol to finally obtain the fraction of cacao bean and cacao bean husk.

Thus obtained extract and fraction of cacao bean and cacao bean husk was investigated for their inhibitory activity against GJIC suppression as well as cancer cell proliferation. The result showed that the extract and fraction of cacao bean and cacao bean husk can not only inhibit DNA synthesis of cancer cells but also inhibit the suppression of GJIC, which is a characteristic phenomenon occurring during the promotion and progression stages of carcinogenesis.

The present invention also relates to a pharmaceutical drug or a food additive which comprise extract or fraction of cacao bean and cacao bean husk as an active ingredient, and the method of manufacturing said pharmaceutical drug or said food additive is according to a known method.

The fraction of cacao bean and cacao bean husk alone can be used as a pharmaceutical drug. However, it can be also combined with pharmaceutically acceptable carriers, forming agents, diluents, etc., to be prepared in the form of powder, granules, capsules and injections.

The cacao bean of the present invention has been used as edible food for a long time and there is no special limitation with respect to the amount of medication of its extract or fraction, but the amount of medication can vary depending on the rate of body absorption, body weight, age, sex, and health condition of a patient, length of administration, method of administration, rate of excretion, the seriousness of illness and the like. In general, it is preferred to administer 0.1-10 mg of the fraction of cacao bean and cacao bean husk per 1 kg of body weight. Therefore, the pharmaceutical drug comprising the active ingredient of the present invention shall be manufactured considering the effective range of the extract or fraction. Thus manufactured unit preparations for administration can be administered according to a specialized medication under the supervision of a specialist or by a patient's request or a few times at regular intervals of time.

The fraction of cacao bean and cacao bean husk can be used as a food additive for drinks, gums, confectioneries and the like. The pharmaceutical drug or a food additive that comprises extract or fraction of cacao bean and cacao bean husk as an active ingredient has an excellent inhibitory effect on prevention of cancer as well as inhibition of cancer development.

Hereunder is given a detailed description of the present invention using the following examples, however, it should not be construed as limiting the scope of the present invention.

### Example 1: Preparation of fraction of cacao bean having an anti-carcinogenic effect

### Step 1. Preparation of an extract having an anti-carcnogenic effect via solvent extraction

Cocoa butter was removed from cacao beans, 6 parts by wt of 50% acetone solution (Duksan Co., Ltd., Korea) was added per 1 part by wt of the remaining fraction (cacao mass) and agitation extraction was performed for 5 hr at 60°C while refluxing. The extract was centrifuged for 30 min at 4°C at the rate of 8,000 rpm (Vision Co., Ltd., Korea) and the resulting supernatant was collected. Extraction was repeated once for the remnant and all the extracts were combined. Thus collected extract was filtered through filter paper (Whatmann 41), evaporated, freeze-dried and finally the extract of cacao bean was obtained.

### Step 2. Preparation of a fraction having an anti-carcinogenic effect via adsorption column chromatography

Cacao bean fraction was prepared via adsorption column chromatography by using the extract prepared in the above step 1 based on the method disclosed in U.S. Pat. No. 6,159,451. More specifically, the extract prepared in the above step 1 was adsorbed to a hydrophobic resin of XAD-4 (Sigma Co., Ltd., U.S.) or HP-20 (Mitsubishi Co., Ltd., Japan) and was fractionated with 20, 40, 60, 80, 100% ethanol subsequently.

### Example 2: Preparation of fraction of cacao bean husk having an anti-carcinogenic effect

### Step 1. Preparation of an extract having an anti-carcnogenic effect via solvent extraction

Cocoa butter was removed from cacao bean husks, 6 parts by wt of 50% acetone solution (Duksan Co., Ltd., Korea) was added per 1 part by wt of the remaining fraction (cacao mass) and agitation extraction was performed for 5 hr at 60°C while refluxing. The extract was centrifuged for 30 min at 4°C at the rate of 8,000 rpm (Vision Co., Ltd., Korea) and the resulting supernatant was collected. Extraction was repeated once for the remnant and all the extract was combined. Thus collected extract was filtered through filter paper (Whatmann 41), evporaed, freeze-dried and finally the extract of cacao bean husk was obtained.

### Step 2. Preparation of a fraction having an anti-carcinogenic effect via adsorption column chromatography

Cacao bean husk fraction was prepared via adsorption column chromatography by using the extract prepared in the above step 1 based on the method disclosed in U.S. Pat. No. 6,159,451. More specifically, the extract prepared in the above step 1 was adsorbed to a hydrophobic filler of XAD-4 (Sigma Co., Ltd., U.S.) or HP-20 (Mitsubishi Co., Ltd., Japan) and was fractionated with 20, 40, 60, 80, 100% ethanol subsequently.

Thus obtained extracts of cacao bean and cacao bean husk, and the subsequently obtained fractions were examined for their anti-carcinogenic effects by means of inhibitory effects on the suppression of GJIC by H₂O₂ and on DNA synthesis of cancer cells, which are characteristic phenomena occurring during the promotion and progression stages of carcinogenesis.

### Example 3: Effect of the extracts and fractions of cacao bean and cacao bean husk on the suppression of GJIC by H₂O₂

The anti-carcinogenic effect of extracts and fractions of cacao bean and cacao bean husk was examined by measuring the GJIC of liver cells according to a known method of Scrape Loading/Dye Transfer (SL/DT) assay (Upham, B. L., Kang, K. S., Cho, H. Y., & Trosko, J. E., *Carcinogenesis,* 18: 37-42, 1997), as further explained below.

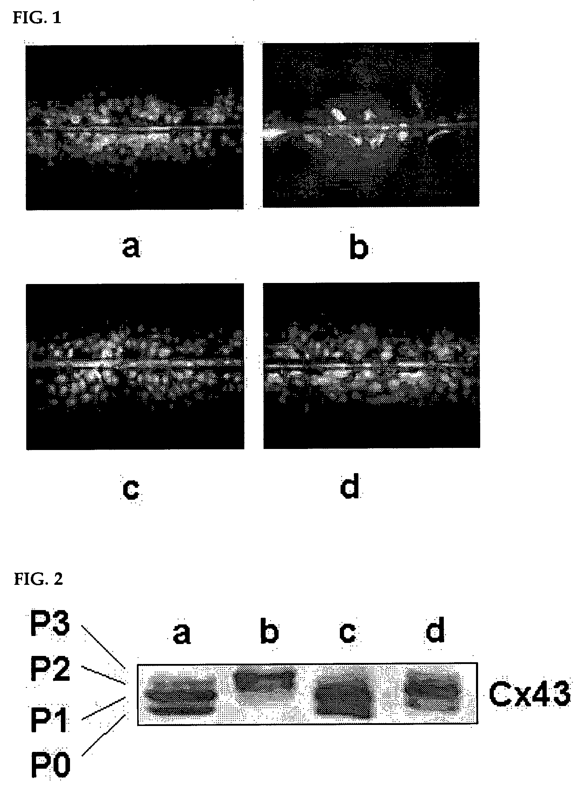

WB-F344 rat liver epithelial cell was used to measure GJIC. WB-F344 was cultured in a MEM media with 10% FBS, added with 100 IU/mL of penicillin and 100 µg/mL of streptomycin, in an incubator at 37 °C with 5% CO₂ (Forma Scientific Co., Marjetta, OH, USA). The media compositions used in the above culture were purchased from GIBCO BRL (Grand Island, NY, U.S.). Thus cultured liver cells were transferred to each 2 mL petri dish (1 x 10⁵ cells/mL) and cultured for 44 hr. Four hours after that, the cells were treated with various concentrations of the extracts and fractions of cacao bean and cacao bean husk with 400 µM H₂O₂, a well-known cancer promoter and one of the strongest reactive oxygen species in the human body, while the control group was also replaced with fresh media only. One hour after the above treatment, the inhibitory level of the extracts and fractions of cacao bean and cacao bean husk on the suppression of gap junction channel by H₂O₂ was observed by means of a confocal microscope (BioRad, Hercules, CA, USA) using Lucifer Yellow dyeing. The effect of the extracts and fractions of cacao bean on the suppression of GJIC is shown in the following Table 1 and FIG.1. The effect of the extracts and fractions of cacao bean husk on the suppression of GJIC is shown in the following Table 2 and FIG.1. The increase rate of GJIC was calculated by measuring the brightness of fluorescence and length revealed in the picture, with reference to a control group of 100% and a group treated with H₂O₂ alone of 0%, wherein activity less than 20% was shown as having almost no activity.

**Table 1**

| **Effect of the extracts and fractions of cacao bean on GJIC increase** | | | | | |
|---|---|---|---|---|---|
| Classification | | GJIC increase rate (%) | | | |
| | | 1 µg/mL | 5 µg/mL | 10µg/mL | 20 µg/mL |
| Extraction solvent | Water | <20 | <20 | <20 | <20 |
| | 50% methanol | <20 | <20 | <20 | 100 |
| | 50% ethanol | <20 | <20 | 50 | 100 |
| | 50% acetone | <20 | <20 | 60 | 100 |
| Solvent fractioning by adsorbing the extract by 50% acetone to styrene-based | Fraction obtained by 20% ethanol | <20 | <20 | <20 | <20 |
| | Fraction obtained by 40% ethanol solvent after fractioning by 20% ethanol | <20 | <20 | 30 | 100 |
| | Fraction obtained by 60% ethanol solvent after fractioning by 40% ethanol | 60 | 100 | 100 | 100 |
| | Fraction obtained by 80% ethanol solvent after fractioning by 60% ethanol | <20 | <20 | 40 | 100 |
| | Fraction obtained by 100% ethanol solvent after fractioning by 80% ethanol | <20 | <20 | <20 | <20 |

**Table 2**

| **Effect of the extracts and fractions of cacao bean husk on GJIC increase** | | | | | |
|---|---|---|---|---|---|
| Classification | | GJIC increase rate (%) | | | |
| | | 1 µg/mL | 5 µg/mL | 10µg/mL | 20 µg/mL |
| Extraction solvent | Water | <20 | <20 | <20 | <20 |
| | 50% methanol | <20 | <20 | <20 | 100 |
| | 50% ethanol | <20 | <20 | 50 | 100 |
| | 50% acetone | <20 | <20 | 30 | 100 |
| Solvent fractioning by adsorbing the extract by 50% | Fraction obtained by 20% ethanol | <20 | <20 | <20 | <20 |
| | Fraction obtained by 40% ethanol solvent after fractioning by 20% ethanol | <20 | <20 | 60 | 100 |
| | Fraction obtained by 60% ethanol solvent after fractioning by 40% ethanol | 70 | 100 | 100 | 100 |
| | Fraction obtained by 80% ethanol solvent after fractioning by 60% ethanol | <20 | <20 | 30 | 100 |
| | Fraction obtained by 100% ethanol solvent after fractioning by 80% ethanol | <20 | <20 | <20 | <20 |

As shown in the above tables 1 and 2, the extracts of both cacao bean and cacao bean husk had a perfect inhibitory effect on the suppression of GJIC by H₂O₂ at concentrations higher than 20 µg/mL. Therefore, it was confirmed that the extracts of both cacao bean and cacao bean husk can prevent cancer by inhibiting GJIC suppression, a characteristic phenomenon in the course of promotion and progression stages of cancer.

Further, the fraction of cacao bean and cacao bean husk obtained by fractionating the extract of cacao bean and cacao bean husk by solvent extraction, filling it into an adsorption resin, fractionating with 40% ethanol and re-fractionating the remnant with 60% ethanol, showed more than 60% of inhibitory effect on GJIC suppression at the concentration of 1 µg/mL while the extracts prepared by 50% acetone and 50% ethanol showed about 30-60% inhibitory effect on GJIC suppression at the concentration of 10 µg/mL, thus proving the excellent anticarcinogenic effects of the fraction of cacao bean and cacao bean husk obtained according to the present invention.

FIG. 1 is a set of pictures that show inhibitory effect of the fraction of cacao bean and cacao bean husk against suppression of GJIC generated by H₂O₂, a cancer promoter and one of the strongest reactive oxygen species (ROS) in the human body, in rat liver epithelial cells (a: a control group; b: a group treated with 400 µM H₂O₂, c: a group treated with 10 µg/mL cacao bean fraction + 400 µM H₂O₂, d: a group treated with 10 µg/mL cacao bean husk fraction + 400 µM H₂O₂).

The inhibitory effect of fraction of cacao bean and cacao bean husk on the hyperphosphorylation of connexin43, a major protein involved in control of gap junction channel, which is regarded as a major phenomenon occurring when GJIC is inhibited by H₂O₂, was also examined by using a known method of a western blot analysis (Upham, B. L., Kang, K. S., Cho, H. Y., & Trosko, J. E. *Carcinogenesis* 18: 37-42, 1997) as further explained below.

Protein was extracted from cells cultured the same as in the GJIC measurement by using 20% SDS which contains 1mM phenylmethylsulfonylfluoride (PMSF). Protein content was measured using a DC assay kit (Bio-Rad Corp., U.S.). About 15 µg each of the extracted proteins was loaded on a 12.5% SDS-PAGE gel and separated by gel electrophoresis. Connexin43 was detected by using an ECL kit (Amersham, Life Science, U.S.) after reacting with a monoclonal body (Zymed, U.S.).

FIG. 2 is a picture that shows inhibitory effect of cacao bean and cacao bean husk on hyperphosphorylation of connexin 43, a major protein that controls GJIC, generated by H₂O₂ in rat liver epithelial cells (a: a control group; b: a group treated with 400 µM H₂O₂, c: a group treated with 10 µg/mL cacao bean fraction + 400 µM H₂O₂, d: a group treated with 10 µg/mL cacao bean husk fraction + 400 µM H₂O₂).

As shown in Tables 1 and 2 and FIGS. 1 and 2, the extracts and fractions of cacao bean and cacao bean husk were shown to have an anti-carcinogenic effect through inhibiting the suppression of GJIC via inhibiting the hyperphosphorylation of Cx43 generated by H₂O₂, a well-known cancer promoter and one of the strongest ROS in the human body, a pathological phenomenon occurring in the carcinogenesis of several cancers including liver cancer.

### Example 4: Inhibitory effect of extracts and fractions of cacao bean or cacao bean husk on cancer cell proliferation (DNA synthesis)

The inhibitory effect of extracts and fractions of cacao bean and cacao bean husk on the DNA synthesis of cancer cells was examined by using ³H thymidine uptake assay (Marshall, E. S. et al. *European J. Cancer,* 30A: 1370-1376, 1994) as further explained below. Examples of cancer cells used were HepG2 as a liver cancer cell, SNU1 as a gastric cancer cell, and SNUC2A as a colon cancer cell.

The HepG2 liver cancer cells, SNU1 gastric cancer cells, SNUC2A colon cancer cells were cultured in RPMI-1640 media with 10% FBS, 100 IU/mL of penicillin and 100 µg/mL of streptomycin, in a 37 °C incubator with 5% CO₂ (Forma Scientific Co., Marjetta, OH, USA) respectively. The media were purchased from GIBCO BRL (Grand Island, NY, USA). Thus cultured cancer cells were transferred to 96 well plates, each of which contains 2 x 10⁴ cells and were added with various concentrations of extracts and fractions of cacao bean and cacao bean husk obtained from the reference examples 1-2 and were cultured for 72 hr. Six hours prior to recovery of the above cells, each well was added with 1 µCi of ³H-Thymidine (Sigma, St. Louis, MO, USA). After completion of the culture, the cells were recovered into a glass fiber filter (Brandel Inc., Gaithersburg, MA, USA) by using a recovery instrument (Cambridge Scientific Inc., cambridge, MA, USA). The incorporation of ³H-thymidine of recovered cells after combining with 3 mL of scintillation cocktail solution (Wallac, Turku, Finland) was examined in a liquid scintillation counter (Wallac, Turku, Finland). The whole process was repeated three times according to the above methods.

Cancer cells were cultured respectively with the extracts and fractions of cacao bean and cacao bean husk at the concentration of 100, 200, 400, 800 µg/mL and then the ³H thymidine uptake assay was carried out. The inhibitory rates of extracts and fractions of cacao bean and cacao bean husk on the proliferation of cancer cells were calculated and shown in the following Tables 3-8.

As shown in the following Tables 3-8, the extracts and the fractions of cacao bean and cacao bean husk obtained by preparing the extract of cacao bean and cacao bean husk by solvent extraction, filling it into an adsorption resin, fractioning with 40% ethanol and re-fractioning the remnant with 60% ethanol, showed excellent inhibitory effect on the proliferation of cancer cell. These fraction of cacao bean and cacao bean husk, being consistent with the fractions of cacao bean and cacao bean husk which had excellent inhibitory effect on the suppression of GJIC as described above, can be used as an excellent anti-carcinogenic agent having both preventive and inhibitory effect in the development of cancer.

| |
|---|
| |

**Table 3**

| **Effect of the extracts and fractions of cacao bean on inhibition of proliferation (DNA synthesis) of liver cancer cells** | | | | | |
|---|---|---|---|---|---|
| Classification | | Inhibition rate of proliferation of liver cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | <20 | 69 |
| | 50% ethanol | <20 | <20 | 37 | 89 |
| | 50% acetone | <20 | <20 | 52 | 93 |
| Solvent fractioning by adsorbing the extract by 50% acetone to styrene-based porous resin | Fraction obtained by 20% ethanol | <20 | <20 | <20 | 43 |
| | Fraction obtained by 40% ethanol solvent after fractioning by 20% ethanol | <20 | <20 | 32 | 76 |
| | Fraction obtained by 60% ethanol solvent after fractioning by 40% ethanol | 68 | 81 | 91 | 95 |
| | Fraction obtained by 80% ethanol solvent after fractioning by 60% ethanol | <20 | <20 | 36 | 73 |
| | Fraction obtained by 100% ethanol solvent after fractioning by 80% ethanol | <20 | <20 | <20 | 32 |

**Table 4**

| **Effect of the extracts and fractions of cacao bean husk on inhibition of proliferation (DNA synthesis) of liver cancer cells** | | | | | |
|---|---|---|---|---|---|
| Classification | | Inhibition rate of proliferation of liver cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | <20 | 79 |
| | 50% ethanol | <20 | <20 | 26 | 89 |
| | 50% acetone | <20 | <20 | 39 | 87 |
| Solvent fractioning by adsorbing the extract by 50% acetone to styrene-based porous resin | Fraction obtained by 20% ethanol | <20 | <20 | <20 | 44 |
| | Fraction obtained by 40% ethanol solvent after fractioning by 20% ethanol | <20 | <20 | <20 | 71 |
| | Fraction obtained by 60% ethanol solvent after fractioning by 40% ethanol | 41 | 79 | 87 | 92 |
| | Fraction obtained by 80% ethanol solvent after fractioning by 60% ethanol | <20 | <20 | <20 | 93 |
| | Fraction obtained by 100% ethanol solvent after fractioning by 80% ethanol | <20 | <20 | <20 | 33 |

**Table 5**

| **Effect of the extracts and fractions of cacao bean on inhibition of proliferation (DNA synthesis) of gastric cancer cells** | | | | | |
|---|---|---|---|---|---|
| Classification | | Inhibition rate of proliferation of gastric cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | <20 | 54 |
| | 50% ethanol | <20 | <20 | 53 | 81 |
| | 50% acetone | <20 | <20 | 49 | 86 |
| Solvent fractioning by adsorbing the extract by 50% acetone to styrene-based porous resin | Fraction obtained by 20% ethanol | <20 | <20 | <20 | 37 |
| | Fraction obtained by 40% ethanol solvent after fractioning by 20% ethanol | <20 | <20 | 68 | 88 |
| | Fraction obtained by 60% ethanol solvent after fractioning by 40% ethanol | 68 | 71 | 92 | 91 |
| | Fraction obtained by 80% ethanol solvent after fractioning by 60% ethanol | <20 | <20 | 43 | 92 |
| | Fraction obtained by 100% ethanol solvent after fractioning by 80% ethanol | <20 | <20 | <20 | 41 |

**Table 6**

| **Effect of the extracts and fractions of cacao bean husk on inhibition of proliferation (DNA synthesis) of gastric cancer cells** | | | | | |
|---|---|---|---|---|---|
| Classification | | Inhibition rate of proliferation of gastric cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | <20 | 68 |
| | 50% ethanol | <20 | <20 | 72 | 79 |
| | 50% acetone | <20 | <20 | 61 | 85 |
| Solvent fractioning by adsorbing the extract by 50% acetone to styrene-based porous resin | Fraction obtained by 20% ethanol | <20 | <20 | <20 | 32 |
| | Fraction obtained by 40% ethanol solvent after fractioning by 20% ethanol | <20 | <20 | 71 | 86 |
| | Fraction obtained by 60% ethanol solvent after fractioning by 40% ethanol | 49 | 76 | 92 | 91 |
| | Fraction obtained by 80% ethanol solvent after fractioning by 60% ethanol | <20 | <20 | 59 | 94 |
| | Fraction obtained by 100% ethanol solvent after fractioning by 80% ethanol | <20 | <20 | <20 | 30 |

**Table 7**

| **Effect of the extracts and fractions of cacao bean on inhibition of proliferation (DNA synthesis) of colon cancer cells** | | | | | |
|---|---|---|---|---|---|
| Classification | | Inhibition rate of proliferation of colon cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | <20 | 71 |
| | 50% ethanol | <20 | <20 | 49 | 83 |
| | 50% acetone | <20 | <20 | 65 | 82 |
| Solvent fractioning by adsorbing the extract by 50% acetone to styrene-based | Fraction obtained by 20% ethanol | <20 | <20 | <20 | <20 |
| | Fraction obtained by 40% ethanol solvent after fractioning by 20% ethanol | <20 | <20 | 52 | 79 |
| | Fraction obtained by 60% ethanol solvent after fractioning by 40% ethanol | <20 | 66 | 89 | 92 |
| | Fraction obtained by 80% ethanol solvent after fractioning by 60% ethanol | <20 | <20 | 45 | 92 |
| | Fraction obtained by 100% ethanol solvent after fractioning by 80% ethanol | <20 | <20 | <20 | 31 |

**Table 8**

| **Effect of the extracts and fractions of cacao bean husk on inhibition of proliferation (DNA synthesis) of colon cancer cells** | | | | | |
|---|---|---|---|---|---|
| Classification | | Inhibition rate of proliferation of colon cancer cells (%) | | | |
| | | 100 µg/mL | 200 µg/mL | 400 µg/mL | 800 µg/mL |
| Extraction solvent | Water | <20 | <20 | <20 | <20 |
| | 50% methanol | <20 | <20 | 28 | 54 |
| | 50% ethanol | <20 | <20 | 44 | 75 |
| | 50% acetone | <20 | <20 | 49 | 88 |
| Solvent fractioning by adsorbing the extract by 50% acetone to styrene-based porous resin | Fraction obtained by 20% ethanol | <20 | <20 | <20 | <20 |
| | Fraction obtained by 40% ethanol solvent after fractioning by 20% ethanol | <20 | <20 | 49 | 57 |
| | Fraction obtained by 60% ethanol solvent after fractioning by 40% ethanol | 32 | 61 | 86 | 91 |
| | Fraction obtained by 80% ethanol solvent after fractioning by 60% ethanol | <20 | <20 | 43 | 88 |
| | Fraction obtained by 100% ethanol solvent after fractioning by 80% ethanol | <20 | <20 | <20 | <20 |

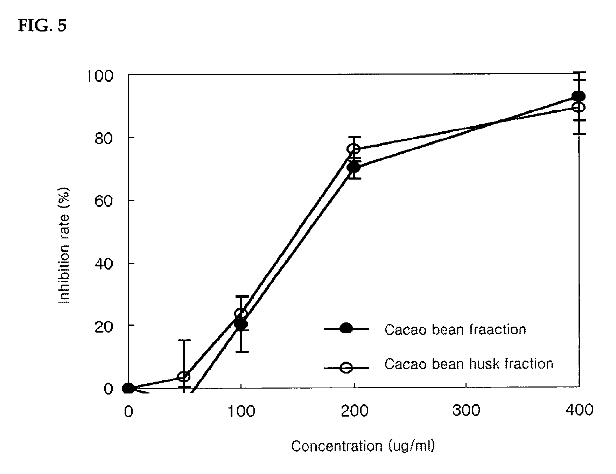

As shown in FIGS. 5-7, the proliferation rates of liver cancer, gastric cancer and colon cancer cells was dependent on the concentrations of fraction of cacao bean and cacao bean husk. Consequently, the fraction of cacao bean and cacao bean husk obtained by preparing the extract of cacao bean and cacao bean husk by solvent extraction, filling it into an adsorption resin, fractioning with 40% ethanol and re-fractioning the remnant with 60% ethanol, showed excellent inhibitory effect on the development of cancer.

### Example 5: Manufacture of Tablets

| | |
|---|---|
| Active ingredient | 10 g |
| Lactose | 70 g |
| Crystalline cellulose | 15 g |
| Magnesium stearate | 5 g |
| Total | 100 g |

The above ingredients were mixed by crushing into small pieces and then prepared into tablets by means of a direct tableting method. Each tablet was prepared to contain total amount of 100 mg and the amount of active ingredient in each tablet was 10 mg.

### Example 6: Manufacture of Powder

| | |
|---|---|
| Active ingredient | 10 g |
| Corn starch | 50 g |
| Carboxy cellulose | 40 g |
| Total | 100 g |

The above ingredients were mixed by crushing into small pieces and then prepared into powder. One hundred gram of powder was filled into each soft capsule to manufacture a final capsule preparation.

### Example 7: Toxicity Test

The extracts and fractions of cacao bean and cacao bean husk were tested for their toxicities as follows: the above extracts and fractions of cacao bean and cacao bean husk were dissolved in 50% aqueous ethanol solution, diluted in water and then administered to rats at the concentration of 1 g/kg and observed for 7 days. The results showed that all the rats were alive.

### Example 8

Chewing gums were manufactured by means of a conventional method by using a composition comprising 20 wt% of gum base, 76.9 wt% of sugar, 1 wt% of flavor, 2 wt% of water and 0.1 wt% of fraction of cacao bean or cacao bean husk obtained from the above Example.

### Example 9

Candies were manufactured by means of a conventional method by using a composition comprising 60 wt% of sugar, 39.8 wt% of starch syrup, 0.1 wt% of flavor and 0.1 wt% of fraction of cacao bean or cacao bean husk obtained from the above Example.

### Example 10

Chewing gums were manufactured by means of a conventional method by using a composition comprising 50 wt% of sugar alcohol, 49.8 wt% of maltose, 0.1 wt% of flavor and 0.1 wt% of fraction of cacao bean or cacao bean husk obtained from the above Example.

### Example 11

Biscuits were manufactured by means of a conventional method by using a composition comprising 25.59 wt% of first grade weak flour, 22.22 wt% of first grade medium flour, 4.80 wt% of sugar, 0.73 wt% of salt, 0.78 wt% of glucose, 11.78 wt% of palm shortening, 1.54 wt% of ammonium bicarbonate, 0.17 wt% of sodium bicarbonate, 0.16 wt% of sodium metabisulfite, 1.45 wt% of rice flour, 0.0001 wt% of vitamin B₁, 0.0001 wt% of vitamin B₂, 0.04 wt% of milk flavor, 20.6998 wt% of water, 1.16 wt% of whole milk powder, 0.29 wt% of dried milk replacer, 0.03 wt% of calcium diphosphate, 0.29 wt% of spray salt, 7.27 wt% of spray milk and 1 wt% of fraction of cacao bean or cacao bean husk obtained from the above Example.

### Example 12

Drinks were manufactured by means of a conventional method by using a composition comprising 0.26 wt% of honey, 0.0002 wt% of thioctic acid amide, 0.0004 wt% of nicotinic acid amide, 0.0001 wt% of riboflavin 5'-phosphate sodium, 0.0001 wt% of pyridoxine HCl, 0.001 wt% of inositol, 0.002 wt% of ortho acid, 98.7362 wt% of water, and 1 wt% of fraction of cacao bean or cacao bean husk obtained from the above Example.

### Example 13

Drinks were manufactured by means of a conventional method by using a composition comprising 3.5 wt% of fruit extract, 4.8 wt% of fruit puree, 7.78 wt% of sugar, 0.11 wt% of citric acid, 82.71 wt% of purified water, and 1 wt% of fraction of cacao bean or cacao bean husk obtained from the above Example.

### Example 14

Sausages were manufactured by means of a conventional method by using a composition comprising 65.18 wt% of pork, 25 wt% of chicken, 3.5 wt% of starch, 1.7 wt% of soybean protein, 1.62 wt% of salt, 0.5 wt% of glucose, 1.5 wt% of glycerine, and 1 wt% of fraction of cacao bean or cacao bean husk obtained from the above Example.

### Example 15

Tablet-type of supplementary health food was manufactured by means of a conventional method by using a composition comprising 55 wt% of spirurina, 10 wt% of enzymetic degradation product of guar gum, 0.01 wt% of vitamin B₁ hydrochloride, 0.01 wt% of vitamin B₆ hydrochloride, 0.23 wt% of DL-methionine, 0.7 wt% of magnesium stearate, 22.2 wt% of lactose, 1.85 wt% of corn starch, and 10 wt% of fraction of cacao bean or cacao bean husk obtained from the above Example.

### Example 16

Capsule-type of supplementary health food was manufactured by means of a conventional method by using a composition comprising 11.26 wt% of chitooligosaccharide, 0.2 wt% of garlic powder, 0.2 wt% of ginkgo extract powder, 0.9 wt% of β-carotene (30% suspension), 1.2 wt% of α-tocopherol, 1.2 wt% of lecithin, 4.5 wt% of refined palm oil, 1.6 wt% of yellow beeswax, 18.994 wt% of soybean oil, 37.83 wt% of gelatin, 16.51 wt% of glycerine, 0.09 wt% of ethylvanilline, 0.076 wt% of titanium dioxide, 0.44 wt% of food color, and 10 wt% of fraction of cacao bean or cacao bean husk obtained from the above Example.

As described above, the extract and fraction of cacao bean and cacao bean husk obtained from the above Examples can be regarded as a food equivalent and thus can be safely administered as an anti-carcinogenic agent without additional purification step to remove toxicity. Further, the process of manufacturing the extract and fraction of cacao bean and cacao bean husk preparation by a simple isolation process enables to reduce unit cost of production as compared to those of conventional anti-carcinogenic agents; in particular, the recycling of huge amount of cacao bean husk which have been discarded as wastes, can be valued much from the economic point of view.

## Claims

1. An anti-carcinogenic agent comprising fraction or extract of cacao bean as an active ingredient.

2. The anti-carcinogenic agent according to claim 1, wherein said cacao bean contains cacao bean husk.

3. A food additive which contains fraction or extract of cacao bean **characterized by** having a cancer-preventive effect as well as a cancer-inhibitory effect.

4. A food additive according to claim 3, wherein said cacao bean contains cacao bean husk.
